# FASCICULE DE BREVET EUROPEEN

(11) **EP 0 730 736 B1**
(45) Date de publication et mention de la délivrance du brevet: **09.07.1997**
(21) Numéro de dépôt: 95902796.2
(22) Date de dépôt: 18.11.1994
(51) Int. Cl.: G01N 33/18

(54) **Procédé et installation pour la surveillance des milieux aqueux par des animaux aquatiques électrogènes**
Verfahren und Einrichtung zur Wasserüberwachung mittels elektrischer Wassertiere
Process and apparatus for monitoring aqueous media using electric aquatic animals

(30) Priorité: 23.11.1993 FR 9314209
(43) Date de publication de la demande: 11.09.1996
(73) Titulaire: CENTRE INTERNATIONAL DE L'EAU DE NANCY ( NAN.C.I.E.), F-54515 Vandoeuvre (FR); UNIVERSITE DE NANCY I, F-54000 Nancy (FR); Florion, André, F-54520 Laxou (FR)
(72) Inventeur: FLORION, André, F-54520 Laxou (FR); TERVER, Denis, F-54600 Villers-lès-Nancy (FR); CHRETIEN, Didier, F-54000 Nancy (FR); THOMAS, Marielle, F-57420 Sologne (FR)
(74) Mandataire: Poupon, Michel
(86) Numéro de dépôt international: FR9401346
(87) Numéro de publication internationale: WO9514925

(56) Documents cités:
- EP-A- 0 158 522
- WO-A-92/04629
- DE-C- 2 906 884
- GB-A- 1 555 683
- US-A- 5 140 855

## Description

L'invention concerne un procédé et une installation pour la surveillance biologique en temps réel de paramètres physico-chimiques des milieux aqueux. utilisant des signaux électriques émis par des animaux aquatiques électrogènes.

Il existe déjà un dispositif de ce type décrit dans le document DE 2 906 884 et permettant de surveiller la qualité de l'eau au moyen d'un poisson électrique placé dans un bac d'observation. L eau du bac est reliée à une électrode pour la réception des décharges du poisson qui sont amplifiées et enregistrées. Une alarme est prévue en cas de dépassement de limites préalablement fixées.

Selon ce document

Le dispositif fonctionne préférentiellement avec des poissons électriques de type pulsatoire irrégulier basse fréquence (*Gnathonemus petersii* et *Mormyrus hasselquisti*) mais également avec une espèce ondulatoire basse fréquence (*Gymnarchus niloticus*).

Il est basé sur la mesure en continu d'un ou plusieurs paramètres non clairement énoncés ; il s'agit soit de l'intensité (on parle de 0 à 5 mA), soit de la tension des signaux (on parle de voltmètre digital), soit de la fréquence (on parle de rythmes nycthéméraux de décharges du poisson). Cette dernière grandeur étant extremement variable pour les poissons considérés, même en l'absence de perturbation (s) environnementale(s), l'exploitation du procédé nécessite un traitement statistique complexe des décharges.

Le dispositif comporte en outre un système de réveil du poisson pour l'empêcher d'observer les périodes de repos diurne pendant lesquelles sa fréquence d'émission diminue Cette activité forcée du poisson entraîne des perturbations de mesure ; en outre elle est néfaste au poisson et n'est donc pas admissible.

L'idée inventive consiste à utiliser des animaux électrogènes ondulatoires haute fréquence et/ou pulsatoires réguliers, en mesurant notamment la fréquence d'émission des décharges ainsi que l'intensité et la forme de leurs signaux. Leur particularité est d'émettre des signaux électriques dont les caractéristiques sont extrêmement stables dans le temps lorsque les conditions environnementales sont elles-memes stables.

Selon un mode de réalisation préféré mais non limitatif de l'invention, on utilise comme animal-test une espèce de poissons appelée *Apteronotus albifrons* de l'ordre des Gymnotiformes.

*Apteronous albifrons* émet un signal ondulatoire de faible amplitude (quelques volts au contact du poisson, quelques millivolts dans les électrodes de captage) et d'une fréquence voisine de 1 000 Hz, extrêmement stable dans le temps (variation de l'ordre de 0,1 %) lorsque les conditions environnementales sont elles-mêmes stables. De plus son signal, loin d'être sinusoïdal, comporte un large spectre d'harmoniques que l'on peut également exploiter pour la bio-surveillance de paramètres physico-chimiques des milieux aqueux.

Ainsi ces poissons tropicaux délivrent continuellement un courant électrique, qu'ils modulent en intensité, en forme et en fréquence suivant divers facteurs de l'environnement tels que la température, le pH, l'oxygène, la présence de substances toxiques,...

En résumé, nous avons pour point de départ la constatation qu'à environnement stable, les caractéristiques du signal électrique sont également stables, et- comme résultat à exploiter le fait que des modifications de facteurs environnementaux entraînent une variation des caractéristiques du signal électrique en terme :
- de fréquence : abaissement, augmentation, pics de fréquence,...
- d'intensité du signal électrique,
- de forme du signal, c'est-à-dire modification du spectre des harmoniques.

L'analyse de ces trois caractéristiques du signal électrique ainsi qu'une étude éventuelle du comportement moteur de l'animal-test par exemple par système vidéo fourniront l'information exploitée par le procédé et l'installation selon l'invention.

Un premier problème à résoudre concerne la dépendance très nette de la fréquence du poisson vis-à-vis de la température de l'eau du bac.

Il s'avère difficile de déterminer précisément les réponses du poisson suite à des variations de facteurs physiques ou chimiques de l'environnement comme par exemple le pH et l'oxygène ou à une présence d'hydrocarbures et d'autres substances toxiques, si la température de l'eau n'est pas maîtrisée parfaitement.

L'objectif de l'invention pour résoudre ce premier problème est de stabiliser la température de l'eau au niveau de l'animal-test pour éviter d'obtenir, dans des conditions standards (en dehors de toute pollution), de brusques variations de la fréquence de l'animal-test de l'ordre de quelques Hertz dans un laps de temps court (hormis les pics de fréquence naturels de l'animal-test).

En effet, étant donné l'étroite dépendance existant entre température et fréquence, il est nécessaire de s'affranchir totalement du facteur température de façon à pouvoir enregistrer et analyser à tout moment une variation d'une ou plusieurs caractéristiques du signal électrique de l'animal-test lors d'une perturbation environnementale.

Un autre problème à résoudre concerne la fréquence du réseau qui peut induire des parasites, ce problème est résolu entre autre par le choix d'une espèce, dont l'espèce citée, qui émet des signaux à hautes fréquences donc nettement différenciables de la fréquence du réseau.

Les objectifs principaux de l'invention sont :
- concevoir un système utilisable en circuit fermé par exemple pour des études expérimentales, ou en circuit ouvert par exemple pour la surveillance en temps réel des eaux de surface ;
- mettre en place un appareillage de thermorégulation performant, capable de maintenir la température de l'eau de l'aquarium la plus constante possible ;
- mettre en place un système permettant d'obtenir une bonne homégénéisation du liquide à surveiller au niveau du bac de contention, c'est-à-dire optimiser :
   . la forme du bac de contention,
   . son volume,
   . le taux de renouvellement de l'eau distribuée dans le bac de contention et dont on veut tester la qualité,
   . le système d'arrivée d'eau dans ce bac.
- pour la détection de toxicité, insérer éventuellement dans l'installation un bac de brassage dans le but d'homogénéiser le milieu ;
- réduire au minimum le volume d'eau circulant en amont du bac de contention pour que le temps de réponse de l'animal-test, mis en présence d'un éventuel polluant, soit le plus petit possible.

Les problèmes cités sont résolus et les objectifs fixés sont atteints grâce au procédé selon l'invention qui utilise les signaux émis par des animaux électrogènes ondulatoires, hautes fréquences et/ou pulsatoires réguliers et plus particulièrement par *Apteronous albifrons.*

Le procédé selon l'invention se caractérise en ce qu'il comporte des étapes successives de stabilisation thermique de l'eau captée, distribution d'eau dans un ou plusieurs bacs de contention contenant chacun un animal-test, traitement des informations avec correction de mesures en fonction d'au moins un paramètre influant sur le signal et n'entrant pas dans le cadre d'une pollution ou d'une perturbation du liquide à surveiller.

De plus, le procédé nécessite l'établissement préalable d'une carte d'identité électrique de l'animal-test pour déterminer les bornes entre lesquelles varient les différentes caractéristiques du signal électrique mesurées dans des conditions standards (milieu non pollué).

De préférence, on utilisera des poissons relais à phase d'activité nycthémérale artificiellement décalée.

L'invention porte également sur toutes les installations qui mettent en oeuvre le procédé précité.

On comprendra mieux l'invention à l'aide de la description qui suit faite en référence aux figures annexées suivantes :
- **figure 1** : schéma de principe du circuit électrique et électronique,
- **figure 2** : schéma de principe d'une première variante du circuit hydraulique,
- **figure 3** : schéma de principe d'une deuxième variante du circuit hydraulique,
- **figure 4** : schéma d'une installation sur site d'une centrale de surveillance de pollution d'eaux de surface,
- **figure 5** : synoptique du programme de traitement.

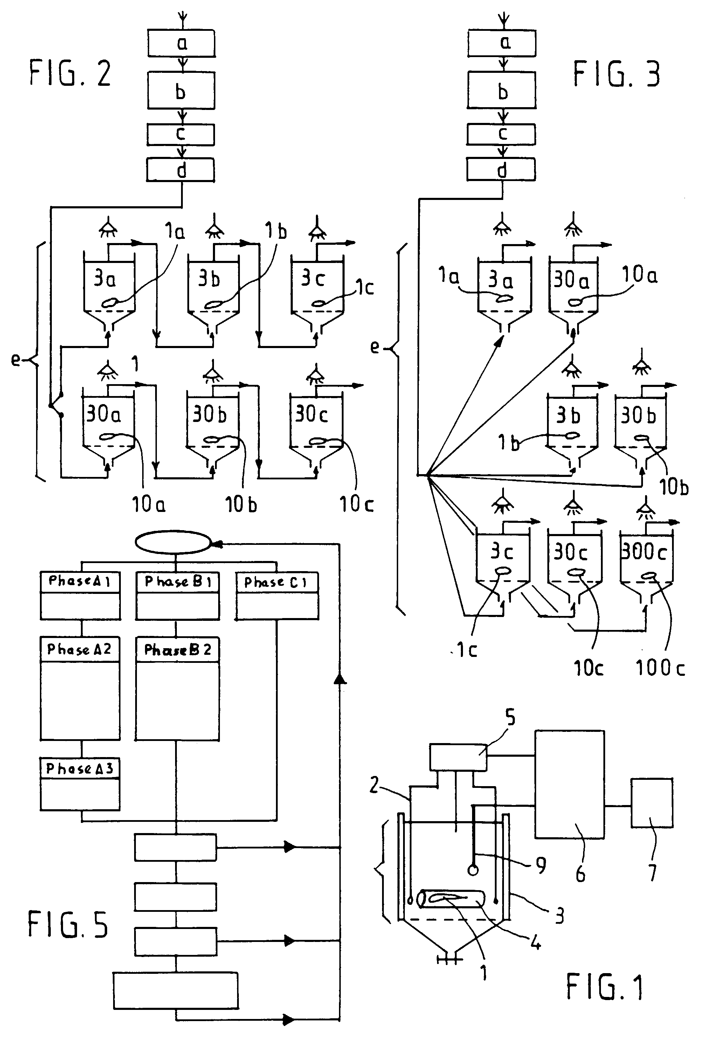

La figure 1 est un schéma de principe du circuit électrique et électronique de l'invention qui comporte les étapes de captage, traitement des informations captées, exploitation des données.

LE CAPTAGE : L'information électrique d' *Apteronotus albifrons* (1) est recueillie par au moins deux électrodes (2) par exemple en inox plongées verticalement dans le plan médian de l'eau d'un aquarium (3). Elles déterminent un canal de captage qui recueille les variations ondulatoires du champ électrique dont s'entoure continuellement l'animal-test (1).

Une sonde thermique (9) détecte la température du bain.

Le captage de l'information électrique est optimal lorsque l'animal-test se tient dans le plan déterminé par les électrodes. Ce problème est résolu par la mise en place à l'endroit voulu d'un refuge (4), par exemple un tube en PVC dans lequel se loge *Apteronotus albifrons*, cette espèce ayant l'habitude dans la nature de se loger dans des cavités ou des trous.

LE TRAITEMENT DE L'INFORMATION : Les variations périodiques ainsi collectées par les électrodes sont faibles (quelques millivolts). Elles font l'objet d'une amplification par exemple par un préamplificateur suivi d'un amplificateur différentiel (5) (avec trois électrodes par exemple) qui porte leur valeur à 12 Volts par exemple, sans modification de leur forme, ni de leur fréquence.

L'EXPLOITATION INFORMATIQUE DU SIGNAL ELECTRIQUE par un micro-ordinateur (6) :
l'enregistrement concerne :
1. La fréquence de l'animal-test : enregistrement à des intervalles de temps définis (de quelques centièmes de secondes à quelques secondes ou minutes) du nombre d'impulsions électriques par seconde.
2. La forme et l'intensité du signal électrique, en particulier, étude des harmoniques (analyse de Fourier).

Le logiciel développé spécifiquement pour cette étude permet la mesure de la fréquence avec une précision de 1/10ème de Hertz. Les mesures se font généralement toutes les secondes (possibilité de travailler sur des intervalles de temps plus grands ou plus petits que la seconde) ; la fréquence est visualisée en temps réel sur un écran (7) et stockée automatiquement dans des fichiers (de 4 heures pour des mesures toutes les secondes) sur une période pratiquement illimitée.

Par ailleurs, le logiciel permet la visualisation en temps pseudo-réel (fonction oscilloscope) de la forme du signal. Toutes les n secondes, un fichier "image" de la courbe peut être stocké sur un disque pour une analyse de Fourier. Par ailleurs, la température de l'eau de l'aquarium où est maintenu l'animal-test est visible à l'écran (7) et stockée en temps réel (précision de l'ordre du 1/100ème de degrés Celsius).

L'analyse des caractéristiques du signal électrique : l'analyse informatique se fait selon le programme dont synoptique en figure 5 (donné à titre d'exemple non limitatif) :

Pour chaque animal, une carte d'identité électrique sera préalablement établie afin de déterminer les bornes entre lesquelles varient les différentes caractéristiques du signal électrique dans des conditions standards (milieu non pollué).

Les signaux sont traités en parallèle selon trois axes A, B, C (voir figure 5) qui se décomposent comme suit :
- phase A1 : Mesure de la fréquence,
- phase A2 : Correction de la mesure en fonction des paramètres mesurés en phase C1,
- phase A3 : Comparaison avec la moyenne glissante des "n" dernières mesures,
- phase B1 : Capture de la forme du signal et analyse spectrale,
- phase B2 : Comparaison avec la "carte d'identité" électrique de l'animal en fonction des paramètres mesurés en phase C1,
- phase C1 : Mesure de la température et éventuellement des paramètres influant sur le signal et n'entrant pas dans le cadre d'une pollution.

Par système de comparaison classique, si les mesures sont normales, ou si une alerte n'est pas confirmée, le programme est réinitialisé, si l'alerte est confirmée, une alarme se déclenche et met en route des procédures variables selon l'origine de l'alerte.

Différents moyens de surveillance et de déclenchement d'alarme peuvent être mis en oeuvre tel que : télésurveillance, téléaction, vidéo.

La figure 2 est un schéma de principe du circuit hydraulique et de régulation thermique pour la mise en oeuvre de l'invention. On distingue les principales étapes suivantes :
a) le prélèvement continuel du liquide dont on veut tester la qualité, au moyen d'une pompe à un débit régulier de façon à renouveler le milieu correctement.
b) selon les situations, étape éventuelle pour l'élimination des particules en suspension. Ceci sera réalisé par exemple par un système de filtration ou par exemple par une décantation lamellaire ; une étape de brassage du liquide à surveiller peut également être intercalée.
c) préchauffage grossier du liquide avec un régulateur thermique fonctionnant en tout ou rien,
d) chauffage très précis du liquide à la température idoine pour l'animal-test (1) (i.e. 26-27°C). Ceci est réalisé au moyen d'une unité thermorégulatrice très précise comportant un régulateur performant, pilotant un gradateur (unité de puissance à thyristors) et d'une sonde platine type Pt 100 située en sortie du réchauffeur, c'est-à-dire dans la conduite entre le bac de chauffage et le bac de contention. Le régulateur délivre un signal variable de 0 à 20 mA au gradateur, correspondant à une régulation variable de la puissance des résistances. Cette unité thermorégulatrice doit permettre une arrivée de liquide dans le bac de contention (3) à une température très stable (de l'ordre de 0,1°C) malgré les écarts thermiques du liquide prélevé au départ.
   Il est important de noter la nécessité d'une alimentation en liquide à température extrêmement constante dans le bac de contention, étant donné l'étroite corrélation existant entre ce facteur et la fréquence de décharges d' *Apteronous albifrons.* En effet, suivant les individus nous enregistrons une variation de 40 à 45 Hz par degré Celsius, ces deux grandeurs variant dans le même sens.
e) distribution du liquide thermorégulé dans un ou plusieurs bacs de contention calorifugés (3). Le liquide thermorégulé est distribué à la base du bac (3) de contention de forme cylindro-conique et est évacué par un dispositif de récupération en surface. L'animal-test (1) est donc soumis à un courant de liquide vertical. La forme cylindro-conique est préférable à la forme parallélépipédique pour une distribution homogène de liquide sur la section entière du bac.

Afin de s'affranchir des variations individuelles des animaux-tests, nous enregistrerons les réponses électriques et comportementales de façon simultanée sur plusieurs animaux par exemple trois animaux (1a, 1b, 1c). Aussi le liquide thermorégulé sera réparti sur trois unités cylindro-coniques (3a, 3b, 3c). Nous envisageons éventuellement un système de bacs-relais, c'est-à-dire un dispositif comprenant deux ou plusieurs groupes de trois bacs. En effet, pour l'animal-test utilisé ici certaines informations électriques sont plus facilement recueillies durant la phase diurne (correspondant à la phase de repos du poisson). Aussi l'idée d'utiliser deux, voire trois animaux-relais, afin qu'il s'en trouve toujours un en phase éclairée, constituera éventuellement un recours intéressant pour une surveillance en continu de la qualité des eaux. Les mesures se feront alors sur le ou les animaux en phase éclairée.

Sur la figure 2 on a représenté un système avec animaux relais comportant deux unités de trois bacs (3a, 3b, 3c) et (30a, 30b, 30c) et deux groupes de trois animaux-tests (1a, 1b, 1c) et (10a, 10b, 10c) dont on décale artificiellement de douze heures les phases diurne et nocture, les mesures se faisant sur le groupe en phase diurne ici le groupe (1a, 1b, 1c), le groupe relais étant en phase nocturne.

A titre d'exemple, la figure 3 montre deux unités de bacs (3a, 30a) et (3b, 30b) comportant chacune deux poissons (1a, 10a) et (1b, 10b) décalés entre eux de douze heures et une unité de bacs (3c, 30c, 300c) comportant trois poissons (1c, 10c, 100c) décalés dans le temps avec des périodes de recouvrement de quatre heures, chaque poisson ayant douze heures de jour et douze heures de nuit.

Bien entendu les nombres d'unités de bacs, ou de bacs dans une unité, ou le nombre d'heures de décalage ou de recouvrement des activités nycthémérales sont choisis en fonction de l'application considérée.

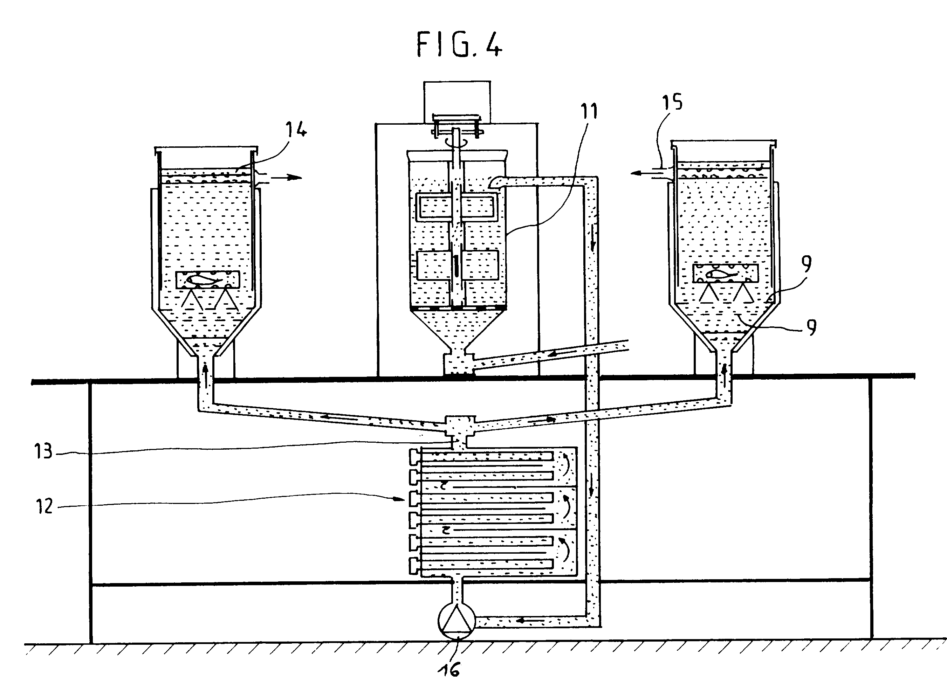

Les bacs peuvent être alimentés en parallèle (figures 3, 4) ou en série (figure 2), dans tous les cas on introduit le liquide en partie basse du bac et on l'évacue en partie haute ceci pour forcer les polluants à faible solubilité et formant un film superficiel tel que les hydrocarbures à traverser les refuges (4).

La figure 4 montre à titre d'exemple non limitatif une installation de mise en oeuvre de l'invention en circuit ouvert. Les eaux de surface prélevées dans le site à surveiller sont envoyées après pompage et filtrage en partie basse d'un bac de brassage (11) classique à pales prévu en amont d'un bac de chauffage (12) pour homogénéiser le milieu.

Le bac de chauffage (12) comporte une pluralité de résistances disposées par exemple horizontalement et en chicanes.

L'eau est portée à une température constante appropriée à la vie des poissons tropicaux (comprise entre 25° et 28°) à partir d'une température du liquide de prélèvement très variable selon la saison.

La sonde qui pilote le thermorégulateur (13) enregistre la température de l'eau à la sortie du bac de chauffage.

Le liquide thermorégulé est envoyé ici dans deux unités de plusieurs bacs de contention (dont deux seulement sont représentés), alimentés en parallèle et disposés en cercle autour du bac de brassage (11).

Chacun des bacs présente ici une section circulaire mais cette forme n'est pas limitative (elle pourrait par exemple être elliptique selon la qualité de captage du signal).

Le fond d'un bac (3) ou (30) comporte une pluralité de plaques perforées (9) pour homogénéiser le flux dans le bac, un refuge (4) en forme de tube perforé dont les perforations permettent d'une part de laisser passer le flux vertical d'autre part d'observer le poisson, un dispositif de prélèvement de liquide (14) en surface avec une conduite (15) pour le rejet sur le site surveillé. Les électrodes (2) sont placées verticalement prés des parois des bacs (3) et dans le plan longitudinal de symétrie du tube (4) pour un captage optimal des signaux.

Une pompe (15) assure un débit constant. Les systèmes de commande, de surveillance, d'alarme électrique et/ou sonore et/ou visuelle, ne sont pas représentés sur les figures.

On peut en outre ajouter selon les besoins :
- un dispositif de surveillance vidéo, une modification comportementale du poisson pouvant constituer une alerte (par exemple si l'animal-test vient piper en surface),
- un dispositif de prélèvement automatique d'échantillons de liquide en cas d'alerte pour analyses,
- un dispositif "eau-claire" pour sauver les poissons en cas de pollution par coupure du circuit hydraulique et branchement automatique sur un circuit d'eau non polluée,
- une unité de surveillance de divers paramètres physico-chimiques (comme pH, oxygène, turbidité, conductivité, ammoniaque, etc...) par des capteurs physico-chimiques spécifiques et renvoyant l'information collectée au niveau de l'ordinateur,
- les performances et avantages de l'invention sont nombreux et notamment :
- sa précision,
- sa rapidité (étude de réponses sublétales),
- sa sensibilité,
- sa fiabilité,
- sa mise en oeuvre aisée,
- son adaptabilité à des applications diverses,
- une approche très complète des caractéristiques du milieu à surveiller (approche biologique, physique et chimique).
- sa capacité de détecter en continu et en temps réel.

Le domaine d'application du procédé ou d'une installation selon l'invention n'est pas limité à la surveillance des eaux de surface en vue de détecter l'apparition d'une pollution, mais il peut être étendu à :
- la surveillance et/ou au contrôle de tous les milieux aqueux dans des domaines industriels variés (par exemple dans des laboratoires pharmaceutiques pour surveiller la stabilité en concentration d'une substance ;
- la detection et/ou la mesure et/ou la régulation de température ou de variations de température nécessitant une précision bien supérieure à celle des appareils connus actuellement, et ce dans n'importe quel domaine industriel (précision du poisson de l'ordre du millième de degré).

Enfin, d'une façon générale l'invention n'est pas limitée à l'utilisation de *Apteronous albifrons* car tous les animaux aquatiques ondulatoires haute fréquence et/ou pulsatoires réguliers peuvent convenir.

## Revendications

1. Procédé pour la surveillance biologique en temps réel de paramètres physico-chimiques des milieux aqueux utilisant des signaux électriques émis par des animaux-tests aquatiques électrogènes caractérisé en ce qu'il utilise les signaux émis par au moins un animal-test aquatique appartenant à l'ensemble constitué par les animaux ondulatoires haute fréquence et les animaux pulsatoires réguliers.

2. Procédé selon la revendication précédente, caractérisé en ce que au moins un des animaux-tests de l'ensemble choisi est un poisson.

3. Procédé selon la revendication 2, caractérisé en ce qu'il utilise au moins un poisson de l'ordre des **Gymnotiformes**.

4. Procédé selon la revendication 3, caractérisé en ce qu'il utilise une espèce animale appelée **Apteronotus albifrons**.

5. Procédé selon l'une quelconque des revendications 1 à 4, caractérisé en ce qu'il comporte des étapes successives de stabilisation thermique du liquide capté, de distribution de liquide dans un ou plusieurs bacs de contention (3) contenant chacun un animal-test (1), de traitement des informations avec correction de mesures en fonction d'au moins un paramètre influant sur le signal.

6. Procédé selon la revendication précédente, caractérisé en ce que le traitement de données nécessite l'établissement préalable d'une carte d'identité électrique de l'animal-test (1) pour déterminer les bornes entre lesquelles varient les différentes caractéristiques du signal électrique dans des conditions standards d'un milieu non pollué.

7. Procédé selon la revendication 6, caractérisé en ce qu'il comporte un programme informatique dans lequel les signaux sont traités en parallèle selon trois axes A, B, C qui se décomposent comme suit :
- phase A1: Mesure de la fréquence,
- phase A2 : Correction de la mesure en fonction d'au moins un paramètre mesuré en phase C1,
- phase A3 : Comparaison avec la moyenne glissante des "n" dernières mesures,
- phase B1 : Capture de la forme du signal et analyse spectrale,
- phase B2 : Comparaison avec la "carte d'identité" électrique de l'animal-test en fonction des paramètres mesurés en phase C1,
- phase C1 : Mesure de la température et éventuellement d'autres paramètres influant sur le signal,
puis, par système de comparaison classique, soit si les mesures sont normales ou si une alerte n'est pas confirmée, le programme est réinitialisé au début, soit si l'alerte est confirmée, une alarme se déclenche et met en route des procédures variables selon l'origine de l'alerte.

8. Procédé selon l'une des revendications précédentes caractérisé en ce qu'on utilise des animaux relais à phase d'activité nycthémérale artificiellement décalée, de façon à ce qu'on ait en permanence au moins un animal-test en phase diurne durant laquelle sont généralement effectuées les mesures.

9. Procédé selon l'une des revendications précédentes caractérisé en ce qu'on enregistre les réponses simultanément sur un groupe d'animaux-tests pendant leur phase de repos.

10. Installation pour la surveillance biologique en temps réel de paramètres physico-chimiques des milieux aqueux utilisant des signaux électriques émis par au moins un animal-test aquatique électrogène comportant :
- un système de traitement des signaux recueillis par les électrodes,
- un système d'exploitation des signaux,
caractérisée en ce qu'elle comporte en outre :
- un système de captage des signaux électriques avec au moins un bac de contention (3) contenant un animal-test (1) appartenant à l'ensemble constitué par les animaux ondulatoires haute fréquence et les animaux pulsatoires réguliers, ledit bac étant équipé d'électrodes recueillant le signal et d'autre part traversé en continu par un flux homogénéisé du liquide à surveiller, et
- une unité thermorégulatrice très précise (12) en amont du ou des bacs de contention (3) pour que les variations de la température dans les bacs de contention n'excèdent pas 0,1°C.

11. Installation selon la revendication 10 caractérisée en ce qu'elle comporte en amont du bac de chauffage (12), un bac de brassage (11).

12. Installation selon l'une des revendications 10 à 11 caractérisée en ce qu'elle comporte au moins deux bacs relais par unité de bacs tels que (3a, 30a) (3b, 30b) (3c, 30c, 300c) dans le but de décaler artificiellement les phases d'activité nycthémérale des animaux-tests entre eux à l'intérieur d'une même unité de bacs.

13. Installation selon l'une des revendications 10 à 11 carcatérisée en ce qu'elle comporte au moins deux unités de bacs tels que (3a, 3b, 3c), (30a, 30b, 30c) dans le but de décaler artificiellement les phases d'activité nycthémérale d'un groupe d'animaux-tests (1a, 1b, 1c) par rapport à l'autre groupe (10a, 10b, 10c).

## Claims

1. Method for the biological surveillance in real time of physico-chemical parameters of aqueous environments, using electrical signals emitted by electrogenic aquatic test animals, characterised in that it utilises the signals emitted by at least one aquatic test animal belonging to the group consisting of high-frequency undulatory animals and regular pulsatory animals.

2. Method according to the preceding claim, characterised in that at least one of the test animals in the chosen group is a fish.

3. Method according to claim 2, characterised in that it utilises at least one fish of the order of **Gymnotiforms**.

4. Method according to claim 3, characterised in that it utilises an animal species called **Apteronotus albifrons**.

5. Method according to any of claims 1 to 4, characterised in that it comprises successive stages of thermal stabilisation of the intercepted liquid, of distribution of liquid in one or more holding tanks (3) each containing a test animal (1), of processing the information with measurement correction in respect of at least one parameter affecting the signal.

6. Method according to the preceding claim, characterised in that the data processing necessitates the prior establishment of an electrical identity card for the test animal (1) in order to determine the limits between which the various characteristics of the electrical signal vary in standard conditions of a non-polluted environment.

7. Method according to claim 6, characterised in that it comprises a computer program in which the signals are processed in parallel according to three axes A, B and C, which break down as follows:
- phase A1 : Measurement of the frequency;
- phase A2 : Correction of the measurement in respect of at least one parameter measured in phase C1;
- phase A3 : Comparison with the continuous mean of the latest "n" measurements;
- phase B1 : Picking-up the shape of the signal and spectral analysis;
- phase B2 : Comparison with the electrical "identity card" of the test animal in respect of the parameters measured in phase C1; and
- phase C1 : Measurement of the temperature and possibly of other parameters affecting the signal,
then, by a conventional comparison system, either, if the measurements are normal or if a warning is not confirmed, the program is started again from the beginning, or, if the warning is confirmed, an alarm is triggered and sets in motion variable procedures depending on the reason for the alarm.

8. Method according to one of the preceding claims, characterised in that a relay of animals is utilised, with an artificially staggered nychthemeral activity phase, so that at least one test animal is permanently available in the diurnal phase during which the measurements are generally effected.

9. Method according to one of the preceding claims, characterised in that the responses are simultaneously recorded from a group of test animals during their resting phase.

10. Installation for the biological surveillance in real time of physico-chemical parameters of aqueous environments, utilising electrical signals emitted by at least one electrogenic aquatic test animal, comprising:
- a system for processing the signals gathered by the electrodes; and
- a system for making use of the signals,
characterised in that it also comprises:
- a system for picking-up the electrical signals having at least one holding tank (3) containing a test animal (1) belonging to the group consisting of high-frequency undulatory animals and regular pulsatory animals, said tank being provided with electrodes gathering the signal and moreover being continuously traversed by a homogenised flow of the liquid to be assessed; and
- a very precise thermoregulatory unit (12) upstream of the holding tank or tanks (3), so that variations in the temperature in the holding tanks do not exceed 0.1° C.

11. Installation according to claim 10, characterised in that it comprises an agitation tank (11) upstream of the heating tank (12).

12. Installation according to one of claims 10 to 11, characterised in that it comprises at least two tanks in relay per unit of tanks such as (3a, 30a) (3b, 30b) (3c, 30c, 300c), with the aim of artificially staggering the phases of nychthemeral activity of the test animals among themselves within the same unit of tanks.

13. Installation according to one of claims 10 to 11, characterised in that it comprises at least two units of tanks such as (3a, 3b, 3c), (30a, 30b, 30c), with the aim of artificially staggering the phases of nychthemeral activity of one group of test animals (1a,1b, 1c) with respect to the other group (10a, 10b, 10c).

## Patentansprüche

1. Verfahren zur biologischen Echtzeit-Überwachung physikalisch-chemischer Parameter wässriger Medien, bei welchem elektrische Signale benutzt werden, welche von elektrogenen Test-Wassertieren abgegeben werden, **dadurch gekennzeichnet**, dass die Signale von zumindest einem Test-Wassertier benutzt werden, welches einer Gruppe angehört, die Tiere mit Hochfrequenzschwingungen und regelmäßige Pulsationen umfasst.

2. Verfahren nach dem vorangegangenen Anspruch, **dadurch gekennzeichnet,** dass zumindest eines der Testtiere der ausgewählten Gruppe ein Fisch ist.

3. Verfahren nach Anspruch 2, **dadurch gekennzeichnet,** dass zumindest ein Fisch der Gattung der Zitteraale (Gymnotiforme) angehört.

4. Verfahren nach Anspruch 3, **dadurch gekennzeichnet,** dass man Tierspezies benutzt, welche Apteronotus albifrons genannt werden.

5. verfahren nach einem der Ansprüche 1 bis 4, **dadurch gekennzeichnet**, dass es aufeinanderfolgend die Schritte der thermischen Stabilisierung der entnommenen Flüssigkeit, der Verteilung der Flüssigkeit in einem Aufnahmebehälter (3) oder mehreren, die jeweils ein Testtier (1) enthalten und den Schritt der Informationsverarbeitung mit einer Berichtigung der Messwerte in Abhängigkeit von zumindest einem Parameter, der auf das Signal einwirkt, aufweist.

6. Verfahren nach dem vorangegangenen Anspruch, **dadurch gekennzeichnet,** dass die Datenverarbeitung zunächst die Erstellung einer elektrischen Identitizierungskarte des Testtieres (1) erforderlich macht, um die Grenzen zu bestimmen, zwischen denen die verschiedenen Kennwerte der elektrischen Signale bei Standardbedingungen eines nicht verunreinigten Mediums schwanken.

7. Verfahren nach Anspruch 6, **dadurch gekennzeichnet,** dass es ein Informationsverarbeitungsprogramm enthält, mit welchem die Signale parallel in drei Achsen A, B, C verarbeitet werden, welche wie folgt gebildet sind,
- Phase A1: Messung der Frequenz,
- Phase A2: Berichtigung der Messung in Abhängigkeit zumindest eines Parameters, gemessen während der Phase C1,
- Phase A3: Vergleich mit dem wechselnden Mittelwert aus "n" der letzten Messungen,
- Phase B1: Gewinnung der Form der Signale und Spektralanalyse,
- Phase B2: Vergleich mit der elektrischen "Identifizierungskarte" des Testtieres in Abhängigkeit der während der Phase C1 gemessenen Parameter,
- Phase C1: Messung der Temperatur und gegebenenfalls weiterer Parameter, welche das Signal beeinflussen,
und dass anschließend mit einem klassischen Vergleichssystem das Programm zunächst reinitialisiert wird, falls die Messwerte normal sind oder eine Warnung nicht bestätigt wurde oder aber für den Fall, dass der Alarm bestätigt, dieser ausgelöst wird und die je nach der Herkunft des Alarm verschiedenen Maßnahmen in Gang gesetzt werden.

8. Verfahren nach einem der vorangehenden Ansprüche, **dadurch gekennzeichnet,** dass man Tiere benutzt, deren nächtliche (nycthemerale) Aktivitätsphase künstlich verschoben wurde, so dass man ständig zumindest ein Testtier in der Tagesphase hat, während der im allgemeinen die Messungen erfolgen.

9. Verfahren nach einem der vorangehenden Ansprüche, **dadurch gekennzeichnet**, dass man gleichzeitig die Reaktionen einer Gruppe von Testtieren während ihrer Ruhephase erfasst.

10. Vorrichtung zur biologischen Echtzeit-Überwachung pysikalisch-chemischer Parameter wässriger Medien, welche elektrische Signale verarbeitet, die von zumindest einem elektrogenen Test-Wassertier ausgehen und welche ein Verarbeitungssystem für von Elektroden aufgenommene Signale und ein Auswertsystem für Signale aufweist, **dadurch gekennzeichnet,** dass es zusätzlich folgendes aufweist:
- Ein Aufnahmesystem für die elektrischen Signale mit zumindest einem Aufnahmebehälter (3), der ein Testtier (1) enthält, das einer Gruppe angehört, die Tiere mit Hochfrequenzschwingungen und regelmäßigen Pulsationen umfasst, wobei der genannte Aufnahmebehälter mit Elektroden zum Aufnehmen des Signals ausgestattet ist und andererseits kontinuierlich von einer homogenen, zu überwachenden Flüssigkeitsströmung durchflossen wird.
- Eine sehr genaue Temperaturregeleinheit (12) strömungsmäßig vor dem Aufnahmebehälter (3) oder den Aufnahmebehältern, so dass die Schwankungen der Temperatur 0,1°C nicht überschreiten.

11. Vorrichtung nach Anspruch 10, **dadurch gekennzeichnet,** dass sie strömungsmäßig vor dem Behälter mit der Heizung (12) einen Rührwerksbehälter (11) aufweist.

12. Vorrichtung nach einem der Ansprüche 10 bis 11, **dadurch gekennzeichnet,** dass sie zumindest zwei zu einer Einheit von Behältern zusammengefasste Behälter (3a, 30a; 3b, 30b; 3c, 30c, 300c) aufweist, um künstlich innerhalb einer Einheit von Behältern die Phase der nächtlichen Aktivität der Testtiere zwischen diesen zu verschieben.

13. Vorrichtung nach einem der Ansprüche 10 bis 11, **dadurch gekennzeichnet,** dass sie zumindest zwei Einheiten von Behältern (3a, 3b, 3c; 30a, 30b, 30c) aufweist, um künstlich die Phase der nächtlichen Aktivität einer Gruppe von der Testtieren (1a, 1b, 1c) in Bezug auf die andere Gruppe (10a, 10b, 10c) zu verschieben.
